(19) Europäisches Patentamt
European Patent Office
Office européen des brevets

(11) **EP 4 011 308 A1**

(12) **EUROPEAN PATENT APPLICATION**

(43) Date of publication:
**15.06.2022   Bulletin 2022/24**

(21) Application number: **20212954.0**

(22) Date of filing: **10.12.2020**

(51) International Patent Classification (IPC):
**A61B 18/02** (2006.01)   **A61B 18/12** (2006.01)
**A61B 18/18** (2006.01)   **A61B 34/00** (2016.01)
**A61B 34/10** (2016.01)   A61B 18/14 (2006.01)
A61B 18/00 (2006.01)

(52) Cooperative Patent Classification (CPC):
**A61B 18/02; A61B 18/12; A61B 18/1815;**
**A61B 34/25;** A61B 2018/00452; A61B 2018/00577;
A61B 2018/00791; A61B 2018/00821;
A61B 2018/0293; A61B 2018/1425;
A61B 2018/1869; A61B 2034/102; A61B 2034/104;
A61B 2034/254; A61B 2034/256

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **TOKOUTSI, Zoi**
  **5656 AE Eindhoven (NL)**

• **BARAGONA, Marco**
  **5656 AE Eindhoven (NL)**
• **FRACKOWIAK, Bruno Jean François**
  **5656 AE Eindhoven (NL)**
• **MAESSEN, Ralph Theodorus Hubertus**
  **5656 AE Eindhoven (NL)**
• **ELEVELT, Aaldert Jan**
  **5656 AE Eindhoven (NL)**

(74) Representative: **Philips Intellectual Property &**
**Standards**
**High Tech Campus 52**
**5656 AG Eindhoven (NL)**

(54) **HEAT DISTRIBUTION MODEL DATABASES FOR PLANNING THERMAL ABLATION**

(57)     A mechanism for adding or updating a heat distribution model stored in a heat distribution model database. A heat distribution model is usable to determine a heat distribution about an ablation device when it is operated, and can be usable to derive thermal profiles in the vicinity of the ablation device. The mechanism comprises obtaining information about fixed properties of the ablation device, generating a heat distribution model based on the fixed properties, and modifying the heat distribution model if, when used, a generated thermal profile is inaccurate.

FIG. 2

EP 4 011 308 A1

**Description**

FIELD OF THE INVENTION

The present invention relates to the field of thermal ablation treatments, and in particular to heat distribution model databases used for planning thermal ablation treatments.

BACKGROUND OF THE INVENTION

Different types or modalities of thermal ablation treatments are known, including microwave (MW), radiofrequency (RF) and cryogenic (cryo) based thermal treatments. A thermal ablation treatment involves using one or more ablation probes or devices to apply thermal damage to a patient in an effort to damage or ablate a target tissue, such as a cancerous growth or a tumor.

Thermal ablation treatments, such as percutaneous ablation treatments, benefit from efficient, effective and accurate treatment planning tools, to plan a desired ablation treatment in advance and adjust the ablation plan (e.g. in real time, during the ablation treatment). Typically, a treatment planning tool will facilitate the determination of an ablation plan that, when executed, is predicted to achieve a desired ablation volume (i.e. ablate a desired volume or zone of a patient, such as area comprising a target tissue). The ablation plan may define the number of ablation probes/devices, their positions within the patient and/or their respective power profiles over time. A more accurate ablation plan, i.e. a plan that achieves the desired ablation volume, improves an outcome of a patient, and avoids damage to surrounding healthy tissue and/or risk regions.

One important factor in accurate treatment planning is to reliably and accurately estimate the thermal profile that results from operating an ablation device.

An existing approach uses the specifications provided by a device manufacturer in order to estimate the thermal damage. Whilst this approach is computationally efficient, it can suffer from inaccuracies in the predicted ablated volume when compared to the "true" or observed ablation volume. The inaccuracies may be due to patient or location (in the patient) specific factors, such as a heat sink effect of blood vessels close to the ablation device, tissue properties that affect the physical process of the ablation and/or the position of a target tissue.

Another approach is to employ a model based treatment planning, where appropriate heat distribution, biophysical and damage models are used to predict the treatment outcome. These models usually involve the modelling of the modality (e.g. MW, RF or cryo) interaction with the tissue and its subsequent heat production, the diffusion of heat in tissue and the resulting thermal damage. The advantage of this approach is that the model parameters can take into account patient-specific tissue properties and geometrical features as well as characteristics of the ablation device. This approach therefore offers the possibility of accurate, personalized treatment planning. However, this approach comes with substantial additional model complexity, which significantly increases computational workload to perform real-time heat distribution modelling.

The model-based treatment planning approach could be modified to use simplified models, which benefits from improved computational cost at the expense of deteriorating the accuracy of the model. There is therefore an ongoing desire to provide simplified models that can be used to accurately predict a thermal profile of an ablation probe/device.

There is a therefore desire to provide accurate heat distribution models for different ablation probes/devices that facilitate improved and more accurate ablation planning and therefore increase a likelihood that an ablation treatment will result in an desired outcome (i.e. a desired ablation zone/volume is correctly ablated).

A suitable method of treatment planning using heat distribution models and the like is provided by International Patent Application No. WO 2019/145211 A1.

SUMMARY OF THE INVENTION

The invention is defined by the claims.

According to examples in accordance with an aspect of the invention, there is provided a computer-implemented method for creating or updating an entry for a target ablation device in a heat distribution model database, each entry mapping an ablation device to a heat distribution model of the ablation device.

The computer-implemented method comprises: obtaining values for one or more fixed properties of the target ablation device; obtaining one or more sample thermal profiles, each sample thermal profile representing the thermal profile that results from operating the target ablation device according to predetermined values of one or more variable parameters of the target ablation device; obtaining, using the values of the one or more fixed properties, a heat distribution model of the target ablation device, the heat distribution model enabling the estimation of a thermal profile that results from operating the target ablation device according to different possible values for one or more variable parameters; for each sample thermal profile, using the heat distribution model and the predetermined values of the one or more variable parameters of the sample thermal profile to generate a predicted thermal profile; determining an accuracy of the heat distribution model by comparing each predicted thermal profile to the corresponding sample thermal profile; in response to the accuracy of the heat distribution model failing to meet predetermined criteria, modifying the heat distribution model; and in response to the accuracy of the heat distribution model meeting predetermined criteria, creating or updating an entry for the target ablation device in the heat distribution model database using the heat distribution model.

The present disclosure proposes a mechanism for providing an improved and adaptable heat distribution model database that can be updated with new ablation devices and/or new information about existing ablation devices. Thus, a heat distribution model database that is more accurate can be provided.

The inventors have recognized that there is a link between highly accurate heat distribution modelling of an ablation device and improved patient outcome. In particular, accurate heat distribution models of an ablation device facilitate more accurate planning, i.e. creation of an ablation plan that, when executed, results in a desired/target volume of a patient being accurately ablated.

A heat distribution model facilitates generation of a heat distribution, and subsequently a thermal profile, for an ablation device (e.g. during an ablation planning process). A heat distribution is a description of the distribution (i.e. size and shape) of heat around an ablation device that results from operating the ablation probe according to values of one or more thermal parameters.

A thermal profile is a description of the distribution of temperature (e.g. temperature values) or damage around an ablation probe that results from operating the ablation probe according to the values of one or more variable parameters. A thermal profile may, for example, indicate a thermal damage (or ablation damage) in the vicinity of the ablation probe (when operated according to the values of the one or more variable parameters), or may indicate a (relative) magnitude of heat output by the ablation device.

More generally, a heat distribution model receives, as input, values for one or more variable parameters of the ablation device (e.g. a length of time the ablation device is operated, a power used by the ablation device, a power profile of the ablation device and so on). The heat distribution model provides, as output, a heat distribution that models the heat distribution provided by the ablation device.

The heat distribution model may comprise, for example, a plurality of sub-models, each sub-model representing a different model of the thermal ablation device for different environments and/or conditions, e.g. for use in different tissue types and/or conditions. Other examples for a heat distribution model will be apparent to the skilled person.

A fixed property of the target ablation device is a property or intrinsic characteristic that is typically not able to be modified during operation of the target ablation device, e.g. a modality of the ablation device, a length of the ablation device or another intrinsic characteristics of the target ablation device.

The proposed mechanism facilitates simple and intuitive addition of a new heat distribution model, or updating of an existing heat distribution model, a heat distribution model database. The proposed mechanism can be used even when there is a sparsity of information about the target ablation device, by automatically modifying a heat distribution model to match known outcomes.

In particular, the proposed method for adding heat distribution models to a database (for use with thermal treatment model/planning software) is an improvement of the current workflow. The proposed mechanism reduces the complexity of the process for including a new target ablation device; reduces the requirements for a user to have expertise or an in-depth understanding of modality specific physics; reduces computational costs by reducing the requirements for dedicated meshing and solvers and uses a simple evaluation of our estimate of the corresponding heat signature. The proposed mechanism also enables the inclusion of ablation devices where very little information is available regarding the design and function of the ablation device and enables a simplified, (semi-)automatic model update/improvement based on newly available information.

In some embodiments, each variable parameter represents either a variable parameter of the target ablation device used when producing the sample thermal profile or a property of an environment in which the target ablation device is operated when producing the sample thermal profile.

The variable parameter may be any parameter that can be modified with respect to a particular target ablation device, e.g. the environment in which the target ablation device is used or operation parameters (e.g. length of time, power, operating mode, configuration and so on) of the target ablation device.

Optionally, the step of obtaining values for one or more fixed properties comprises obtaining a user input defining values for at least one of the one or more fixed properties; and/or the step of obtaining one or more sample thermal profiles comprises obtaining one or more sample thermal profile not previously associated with the target ablation device.

In some embodiments, the step of generating a heat distribution model for the target ablation device comprises: processing the heat distribution model database using the values of the one or more fixed properties of the target ablation model to identify a similar heat distribution model, the similar heat distribution model having similar values for the one or more fixed properties as the corresponding values of the target ablation model; and using the heat distribution model of the similar target model as the heat distribution model for the target ablation device.

Optionally, the step of generating a heat distribution model for the target ablation device comprises: obtaining a heat distribution sub-model database, each entry in the heat distribution sub-model database providing a heat distribution sub-model for different values of one or more potential fixed properties for an ablation device; processing the heat distribution sub-model database using the values of the one or more fixed properties of the target ablation model to identify one or more heat distribution sub-models for the target ablation device; and processing the identified one or more heat distribution sub-models to generate a heat distribution model for the target ablation device.

In at least one embodiment, the one or more variable parameters of the target ablation device comprise: a length of operation of the target ablation device; a power provided to the ablation device; a power profile of the target ablation device over time; a position of the target ablation device within a subject; a tissue type surrounding the target ablation device during operation of the target ablation device; a tissue condition of the target ablation device during operation of the target ablation device; an operating mode of the target ablation device.

Optionally, wherein the step of, in response to the accuracy of the heat distribution model failing to meet predetermined criteria, modifying the heat distribution model comprises performing iterative steps of: modifying the heat distribution model; for each sample thermal profile, using the modified heat distribution model and the predetermined values of the one or more variable parameters of the sample thermal profile to regenerate a predicted thermal profile; and determining an accuracy of the heat distribution model by comparing each predicted thermal profile to the corresponding sample thermal profile, wherein the iterative steps are performed until the accuracy of the heat distribution model meets the predetermined criteria.

In some embodiments, the step of modifying the heat distribution model comprises using an optimization algorithm, such as a least squares fitting algorithm, a genetic algorithm, a machine-learning approach, a gradient descent approach and/or a combinational optimization approach to iteratively modify the heat distribution model until the accuracy of the heat distribution model meets the predetermined criteria. The above described approaches facilitate automated tuning or modifying to the heat distribution model to improve its accuracy.

Optionally, the heat distribution model comprises one or more thermal functions that estimate a distribution of heat about the target ablation device during operation of the target ablation device based on one or more variable parameters.

In some examples, the step of obtaining one or more sample thermal profiles comprises obtaining one or more sample thermal profiles generated by using the target ablation device in an experimental set up to generate one or more sample thermal profiles for different values of the one or more variable parameters.

The method may further comprise step of performing a sensitivity analysis on the obtained heat distribution model. The step of modifying the heat distribution model may be dependent upon an outcome of the sensitivity analysis.

There is also proposed a method of generating an ablation plan for a subject, comprising generating an ablation plan for a subject using a heat distribution model database comprising at least one entry created or updated using the method previously described.

There is also proposed a computer program product comprising computer program code means which, when executed on a computing device having a processing system, cause the processing system to perform all of the steps of any herein described method.

There is also proposed a processing system for creating or updating an entry for a target ablation device in a heat distribution model database, each entry mapping an ablation device to a heat distribution model of the ablation device.

The processing system is configured to: obtain values for one or more fixed properties of the target ablation device; obtain one or more sample thermal profiles, each sample thermal profile representing the thermal profile that results from operating the target ablation device according to predetermined values of one or more variable parameters of the target ablation device; generate, using the values of the one or more fixed properties, a heat distribution model of the target ablation device, the heat distribution model enabling the estimation of a thermal profile that results from operating the target ablation device according to different values for one or more variable parameters; for each sample thermal profile, using the heat distribution model and the predetermined values of the one or more variable parameters of the sample thermal profile to generate a predicted thermal profile; determine an accuracy of the heat distribution model by comparing each predicted thermal profile to the corresponding sample thermal profile; in response to the accuracy of the heat distribution model failing to meet predetermined criteria, modify the heat distribution model; and in response to the accuracy of the heat distribution model meeting predetermined criteria, create or update an entry for the target ablation device in the heat distribution model database using the heat distribution model.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an ablation device;
Fig. 2 illustrates a mechanism for generating a thermal profile for an ablation device;
Fig. 3 illustrates a method according to an embodiment;
Fig. 4 illustrates an ablation device having one or more visible fixed properties;
Fig. ig 5 illustrates a heat distribution model for an ablation device;
Figs. 6 and 7 illustrates an experimental setup for determining sample thermal profiles for use in an embodiment;
Fig. 8 illustrates a method according to an embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for adding or updating a heat distribution model stored in a heat distribution model database. A heat distribution model is usable to determine a heat distribution about an ablation device when it is operated, and can be usable to derive thermal profiles in the vicinity of the ablation device. The mechanism comprises obtaining information about fixed properties of the ablation device, generating a heat distribution model based on the fixed properties, and modifying the heat distribution model if, when used, a generated thermal profile is inaccurate.

The present disclosure relies on the underlying recognition that an accuracy of heat distribution models can be improved by using sample thermal profiles and new/additional information about the ablation device to improve the modelling of heat distribution about the ablation device.

Embodiments of the invention may be employed to generate/update heat distribution models for use in ablation planning tools and/or software.

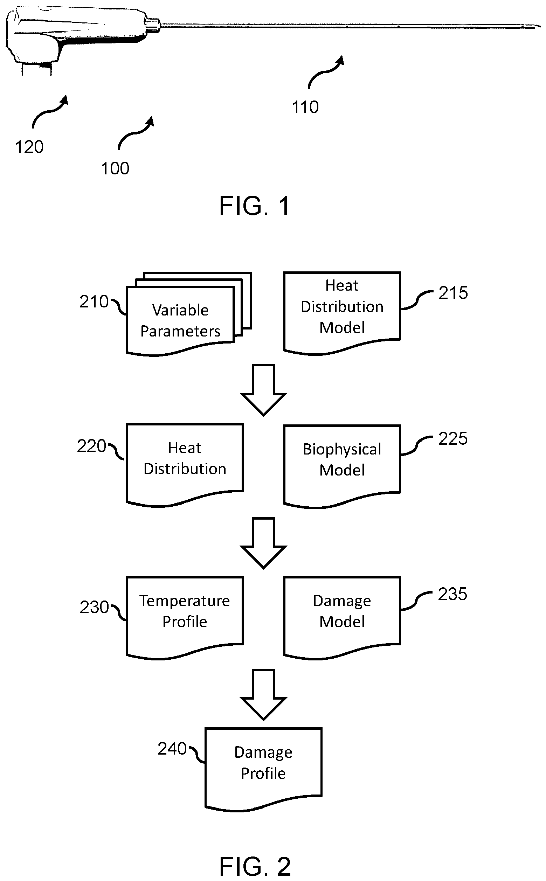

Fig. 1 illustrates an ablation device 100 for the purposes of improved contextual understanding of the use case for the present disclosure. The illustrated ablation device 100 is a microwave (MW) ablation device, configured to receive electric current and output heat or thermal energy, in the form of microwaves, for irradiating or damaging tissue in the vicinity of the ablation device.

The ablation device 100 is formed of a probe 110 and a control element 120. The control element 120 may extend beyond the visible components of the ablation device 100 (e.g. including a separate ablation control unit disposed apart from the control element).

The probe 110 contains one or more antennas (not visible) that, when current is applied, output a microwave or microwaves for ablating or heating an area of proximate tissue. The magnitude of the microwave(s) is dependent upon a magnitude of the current flowing through the antenna(s).

The control element 120 controls the current flowing to the antenna(s), to thereby control the magnitude by the microwave ablation device 110. The control element 120 may also control a cooling of the probe 110, e.g. control a supply of cooling gas or liquid provided along the axis of the probe.

The skilled person will appreciate that different parameters / properties / characteristics of the ablation device 100, and the tissue in which the ablation device is operated, will affect the magnitude, shape and size of temperature changes to the tissue and/or damage to the tissue (i.e. the thermal profile) that results from operating the ablation device (i.e. within the patient).

These parameters / properties / characteristics can either be fixed or non-variable (such as a shape, type, configuration, material, position or other fixed property of (an element of) the ablation device) or variable/non-fixed (such as power applied (by the control element), length of time the ablation device is powered or the location within the patient/tissue) .

There is a strong desire to accurately predict the thermal profile that results from operating the ablation device. This facilitates improved and more accurate ablation planning, thereby leading to a reduced likelihood that sensitive or non-desired tissue is ablated, and that desired tissue is fully ablated (i.e. that a desired/intended thermal profile for an ablation plan correctly reflects the real-life thermal profile that results from executing the plan).

In the context of the present disclosure, a "thermal profile" may be a temperature distribution and/or a damage distribution around the ablation device, when it is operated in tissue according to some values of variable parameters for the ablation device. A temperature distribution identifies the (change in) temperature in different locations of the tissue surrounding the ablation device. A damage distribution identifies a damage to the tissue at different locations surrounding the ablation device.

Fig. 2 illustrates a mechanism 200 by which a thermal profile can be predicted from information about the thermal ablation device and the tissue in which the thermal ablation device is operated.

A thermal profile 230, 240 can be calculated using a heat distribution model 215 (specific to a particular ablation device) and a biophysical model 225. Optionally, a damage model 235 may also be used.

The heat distribution model 215 is used to map variable parameters 210 of the ablation device to the heat distribution 220 or "heat signature" that results from operating the ablation device according to said parameters. For example, the heat distribution model 215 may comprise one or more thermal functions that estimate a distribution of heat about the target ablation device during operation of the target ablation device based on one or more variable parameters.

The heat distribution model 215 can thereby predict the size/extent and shape of heat output by the ablation device. In other words, it may determine the distribution (i.e. size and shape) of heat that results from operating an ablation device according to certain variable parameters of the ablation device.

The heat distribution or heat signature may be tissue-specific. To this end, the heat distribution model 215 used to generate the heat distribution may be tissue specific, or may employ tissue information when generating the heat distribution or heat signature. This concept appreciates how a heat distribution about an ablation device may be tissue-specific and facilitates more accurate modelling or prediction of the thermal profile.

However, in other example, the heat distribution model 215 may be tissue non-specific, i.e. generic. This facilitates a simpler and less resource-intensive, but potentially less accurate, mechanism for predicting the thermal profile.

It will be clear that different values for variable parameters 210 of the ablation device would affect the shape and size (i.e. distribution) of heat output by the ablation device. For example, operating an ablation device at a first power level will result in a different heat distribution to operating the ablation device at a second, different power level (assuming all other variables remain constant).

The resulting heat distribution 220 or heat signature can be further processed, e.g. using a biophysical model 225 and optionally damage model 235 (such as the Arrhenius model), to predict a thermal profile 230, 240 (such as a temperature distribution 230, damage distribution 240, temperature profile 230 or damage profile 240).

A biophysical model may, for example, describe the diffusion of heat in living tissue, thereby enabling a temperature distribution 230 (or temperature profile) around the ablation device within the living tissue to be predicted. A temperature distribution may define or predict the actual temperature of different areas of the tissue (rather than simply determining a heat distribution).

Suitable examples of biophysical models will be apparent to the skilled person. Equation 1 illustrates one known example of a biophysical model:

$$\rho C_p(T)\,\partial_t T - \nabla k(T)\nabla T + \rho_{bl}C_{p,bl}w_{bl}(T)(T - T_{core}) = Q \qquad (1)$$

where Q is the heat distribution, $\rho$, $\rho_{bl}$ is the tissue and blood density respectively, k is the thermal conductivity, $C_p$ and $C_{p,bl}$ is the specific heat capacity of the tissue and of blood respectively, $w_{bl}$ is the blood perfusion rate and $T_{core}$ = 37 degC is the core body temperature. Equaiton (1) enables the calculation of a temperature T.

This temperature distribution 230 may be further processed using a damage model 235 (e.g. the Arrhenius model or the equivalent-minutes formulation of Sapareto and Dewey) to predict a distribution of tissue damage surrounding the ablation device - a damage profile or "damage distribution" 240.

For example, the Arrhenius model can be applied using the approach set out in F.C. Henriques and A.R. Moritz. "Studies of thermal injury: I. the conduction of heat to and through skin and the temperatures attained therein", The American journal of pathology, 23(4):530-549, 1947 or A.R. Moritz and F.C. Henriques. "Studies of thermal injury: II. the relative importance of time and surface temperature in the causation of cutaneous burns", The American journal of pathology, 23(5):695--720, 1947.

Sapareto and Dewey described an equivalent-minutes approach in their paper Sapareto, Stephen A., and William C. Dewey. "Thermal dose determination in cancer therapy." International Journal of Radiation Oncology Biology Physics 10.6 (1984): 787-800.

The skilled person would appreciate how predicted thermal profiles 230, 240 can be used for (automatic) planning of an ablation procedure, i.e. generating an ablation plan. It is therefore important for predicted thermal profile to be of high accuracy.

The present disclosure provides mechanisms for improving the accuracy of a heat distribution model, used to predict the shape/size (i.e. distribution) of heat around the ablation device, stored in a heat distribution model database for target ablation device. In particular, the present disclosure provides an easy to use mechanism for semi-automatically creating and/or updating a heat distribution model for a target ablation device stored in a heat distribution model database.

A database or databank of accurate heat distribution models facilitates increased ease and reduced complexity in generating thermal profiles. The inventors have recognized that a highly accurate heat distribution models would increase an accuracy of an ablation plan, thereby leading to improved patient outcomes.

The present disclosure provides mechanisms for improving the accuracy of heat distribution models within a heat distribution model database. This facilitates improved, and more accurate, predictions of thermal profiles for an ablation device and thereby more accurate ablation planning.

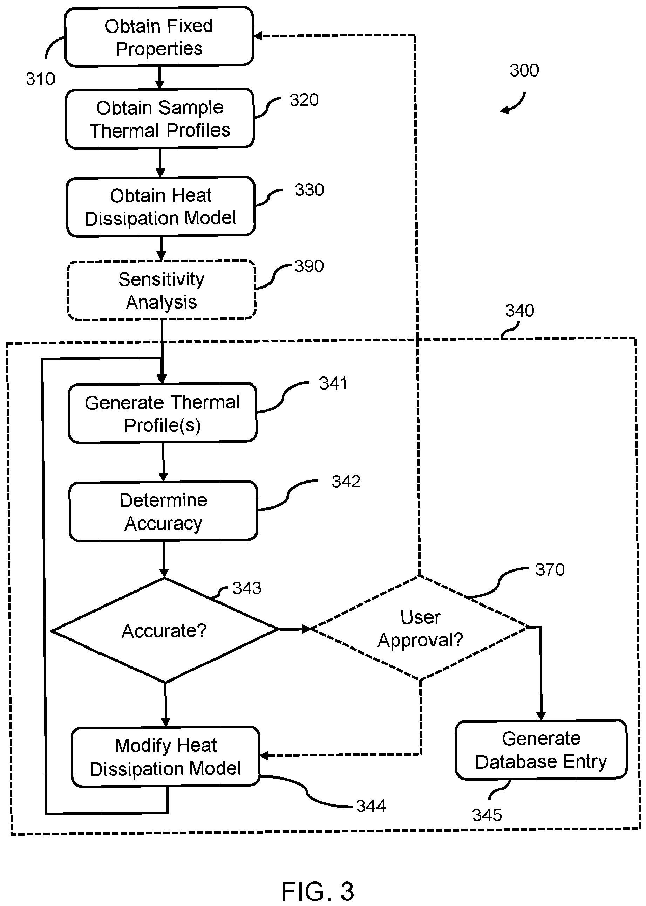

Fig. 3 provides an overview of a process 300 according to an embodiment of the invention. The process 300 provides a mechanism for creating or updating an entry in a heat distribution model database for a target ablation device. The heat distribution model database may be stored in a storage unit or memory (not illustrated).

The process 300 comprises a step 310 of obtaining values for one or more fixed properties of a target ablation device. The fixed properties are properties of the ablation device that are not changed during an operation of the ablation device, examples of which are described throughout this disclosure.

Preferably, step 310 comprises receiving information about the ablation device from an external source, e.g. a user input (via a user interface) and/or a secondary database. For example, a user input may indicate an existing ablation device within the heat distribution model database to identify the fixed properties of the target ablation device. As another example, e.g. where the target ablation device is previously known to the heat distribution data base, the user input and/or secondary database may provide information of a plurality of different fixed properties of the ablation device.

The process 300 further comprises a step 320 of obtaining one or more sample thermal profiles. Each sample thermal profile represents a thermal profile (e.g. temperature or damage distribution) that results from operating the target ablation device according to predetermined values of one or more variable parameters of the target ablation device.

Where the target ablation device is previously unknown to the heat distribution model database, the sample thermal profiles may be provided by an external source, such as a user input (via a user interface) or a secondary database.

In particular, sample thermal profiles are commonly made available by a manufacturer of an ablation device (e.g. included in documentation accompanying the ablation device). Thus, sample thermal profiles may be obtained from documentation of an ablation device.

Purely by way of example, step 320 may comprise obtaining an image illustrating a sample thermal profile, such as an image of an ablation zone performed on tissue, and processing the image to obtain a sample thermal profile. The image may be provided by a user via a camera of a user interface, such a cellphone camera. It is recognized that (physical/paper) documentation of an ablation device may provide information on the sample thermal profiles for that ablation deice. This information can be exploited to provide sample thermal profiles for use with the proposed mechanism.

Where the target ablation device is known to the heat distribution model database, the sample thermal profiles may comprise sample thermal profiles previously used to generate the existing heat distribution model for the target ablation device. These sample thermal profiles may be stored by the heat distribution model database, or another auxiliary storage unit.

Another possible method of obtaining a sample thermal profile is obtaining a sample thermal profile generated during an experimental process, which will be described below.

A variable parameter of the target ablation device may be a variable parameter used when producing the sample thermal profile or a property of an environment in which the target ablation device is operated when producing the sample thermal profile. Preferably, at least one sample thermal profile is not previously associated with the target ablation device

The process then moves to a step 330 of obtaining, using the values of the one or more fixed properties, a heat distribution model of the target ablation device.

Various embodiments for step 330 are envisaged in the present disclosure.

In a first scenario, step 330 comprises identifying a different ablation device, having a heat distribution model stored in the heat distribution model database, that is similar to the target ablation device. This may be performed (semi-)automatically, e.g. using a nearest neighbor mechanism or the like, to identify a different ablation device that shares (the most) similar fixed properties to the target ablation device, and identifying the heat distribution model of the different ablation property.

In a second scenario, step 330 comprises processing the fixed properties to generate a heat distribution model based on known characteristics of the fixed properties, e.g. according to literature and/or prior research. For example, certain elements of the fixed properties (e.g. a particular shape of the tip of the ablation device or a modality of the ablation device) may by itself have a known heat distribution model or known physical properties, which can contribute to the overall heat distribution model of the ablation device.

In the second scenario, step 330 may comprise obtaining one or more sub-models of heat distribution, and processing the one or more sub-models to generate the heat distribution model. For example, each sub-model may be associated with a different value of a fixed property of the ablation device (e.g. type of antenna for a MW ablation device or a hotspot of a MW ablation device). Each sub-model may model the heat distribution for that particular element. The sub-models may be combined, e.g. superimposed on one another or multiplied together, to generate or obtain the (overall) heat distribution model.

By way of further explanation, an ablation device may comprise multiple ablation elements or cooling elements (e.g. multiple elements that output or otherwise affect energy or heat). A heat distribution sub-model may be obtained for each separate ablation/cooling element and combined to form the heat distribution model. In other words, an overall shape and size of heat provided by an ablation device can be determined by combining shapes that represent different features of the ablation device.

The skilled person would readily envisage multiple mechanisms for combining sub-models to form an overall heat distribution model, e.g. superimposing the sub-models, cumulatively applying the sub-models, modelling an interaction between different sub-models and so on.

The sub-models may be stored by a heat distribution sub-model database, each entry in the heat distribution sub-model database providing a heat distribution sub-model for different values of one or more potential fixed properties for an ablation device. Step 330 may comprise obtaining such a database and processing it, based on the fixed properties of the target ablation device, to identify one or more sub-models for the target ablation device (which are then processed to generate the heat distribution model).

In some embodiments, step 330 may employ standard or simple functions, such as Gaussian, sigmoid, rational, and other functions to represent the expected heat distribution about the target ablation device. The parameters for the heat distribution model (e.g. these standard or simple functions) may be adjusted (e.g. based on literature or known characteristics of fixed properties) to generate or obtain the heat distribution model for the target ablation device.

In yet other embodiments, where the target ablation device is known to the heat distribution model database, step 330 may comprise simply obtaining the corresponding heat distribution model from the heat distribution model database, e.g. based on an identity of the target ablation device (a simple example of a fixed property).

Step 330 may comprise determining a range of permissible values for one or more parameters of the heat distribution model. The range of permissible values may be defined by permissible or theoretical limits for the parameters of said heat distribution model (e.g. a maximum or minimum possible extent of a thermal interaction). As will be later described, the results of a sensitivity analysis may also/otherwise be used to limit the bounds of any modification made to the heat distribution model.

Step 330 may further comprise obtaining or receiving a user input. A user may be able to enhance or improve the generation of the (initial) heat distribution model, e.g. by selecting one of an appropriate range of possible initial heat distribution models as the (initial) heat distribution model for the target ablation device.

Thus, for example, step 330 may comprise identifying a plurality of possible heat distribution models, which are presented to a user via a user interface. The user may then select a heat distribution model for further processing by the rest of the method. This enables a user to provide their insight on the heat distribution model.

The process 300 then performs a process 340 of iteratively modifying (if necessary) the heat distribution model.

This is performed by performing a step 341 of, for each sample thermal profile, using the heat distribution model and the predetermined values of the one or more variable parameters of the sample thermal profile to generate a predicted thermal profile.

In particular step 341 generates, for each sample thermal profile, a predicted thermal profile using the same values of the one or more variables that were used when generating the sample thermal profile.

Step 341 may be performed, for example, by using the heat distribution model to predict a heat distribution of the ablation device, and then using a generic biophysical/biological model, and optionally a damage model, to further process the predicted heat distribution to generate the predicted thermal profile. Methods of generating a thermal profile have been previously described.

In particular, step 341 may use the values of the variable parameters used to produce the sample thermal profile to generate the predicted thermal profile. Thus, the sample thermal profile may be associated with certain variable parameters (e.g. power input, time of ablation, tissue information and so on) that act as inputs to the heat distribution model for generating the predicted thermal profile. If one or more values of possible variable parameter for the sample thermal profile are not known, then default values may be used.

Subsequently, the process 340 performs a step 342 of determining an accuracy of the heat distribution model by comparing each predicted thermal profile to the corresponding sample thermal profile.

The accuracy may be a numerical measure of the accuracy of the heat distribution model, so that determining an accuracy comprises determining a numerical measure of the accuracy. However, other suitable measures, e.g. binary or categorical measures, will be apparent to the skilled person.

Step 342 may comprise determining a numerical measure of a difference between each sample thermal profile and the corresponding predicted thermal profile. If multiple numerical measures are generated (e.g. there are multiple thermal samples), then the numerical measures may be further processed to determine a single numerical measure indicative of an accuracy of the heat distribution model. This further processing may comprise averaging the numerical measures or selecting a maximum of the numerical measures as indicative of the accuracy of the heat distribution model.

Mechanisms for determining an accuracy or error value between two thermal profiles will be apparent to the skilled person, for example, using a root-mean-squared deviation or a mean absolute error.

The process 340 determines, in a step 343 whether the accuracy of the heat distribution model meets predetermined criteria. For example, where the accuracy is a numerical measure, step 343 may comprise determining whether or not the numerical measure breaches a predetermined threshold.

In response to the accuracy of the heat distribution model failing to meet predetermined criteria, the process performs a step 344 of modifying the heat distribution model.

In response to the accuracy of the heat distribution model meeting predetermined criteria, the process 340 performs a step 345 of creating an entry for the target ablation device in the heat distribution model database using the heat distribution model. Thus, step 345 adds the heat distribution model to an entry the heat distribution model database, which enables the target ablation device to be linked to the heat distribution model (for later use, e.g. for an ablation planning process). The process 340 then ends.

Process 340 effectively describes a generalized form of an iterative modification approach, in which the heat distribution model is iteratively modified until the accuracy of the heat distribution model meets some predetermined criteria. Thus, process 340 may use an optimization or minimization approach to iteratively modify the heat distribution model.

The iterative modification may be performed, for example, using an optimization algorithm, such as a least squares fitting algorithm, a genetic algorithm, a machine-learning approach, a gradient descent approach, surrogate models, stochastic optimization, and/or a combinational optimization approach. Other approaches for automatically and iteratively modifying a model will be apparent to the skilled person. The iterative modifications are performed until the accuracy of the heat distribution model meets the predetermined criteria.

Thus, process 340 may employ an optimization algorithm, such as any above described iterative modification approach that fits the parameters of the heat distribution model to minimize the difference in the predicted thermal profile from the documented thermal profile. Process 340 may effectively be replaced by a single process of performing an optimization or parameter fitting process to the heat distribution model.

As a further example, process 340 may employ a genetic algorithm (GA) to locate multiple parameter combinations (for the heat distribution model) that produce a similar outcome. This is of particular use in the case where multiple parameter combinations produce predicted ablations that have the same deviation from the true ablation.

As yet another example, an appropriate machine learning approach may be used to modify the heat distribution model. Example machine learning approaches include gradient descent, particle swarm optimization or simulated annealing.

A combinatorial optimization approach may be appropriate in the case were the best fitting heat source has to be chosen from a precomputed database.

In particular examples of the invention, step 345 (of creating a database entry) is only performed if a user has indicated acceptance of the heat distribution model, e.g. via a user input provided via a user interface.

There may therefore be an additional determining step 370, between steps 343 and 345, which comprises determining whether or not a user input (signal) indicates acceptance of the heat distribution model.

To aid the user in making a decision as to whether to accept the heat distribution model, example heat distributions and/or thermal profiles generated using the heat distribution model may be displayed to the user, e.g. at a user interface.

If a user accepts the heat distribution model, step 345 may be performed.

If a user does not accept the heat distribution model, the process 340 may be repeated (e.g. with modified accuracy criteria for step 343 and/or additional user-provided information). In particular, a user may be able to provide additional information (e.g. manually adjust one or more parameters of the heat distribution model) that can be used to improve the optimization/modification process 340.

In another example, if a user does not accept the heat distribution model, the process 300 may be repeated from the beginning (e.g. with additional user-provided information).

From the foregoing, it will be apparent that the process of determining an accuracy of the heat distribution model may further comprise determining a user-indicated accuracy of the heat distribution model.

In some embodiments of the invention, before performing the process 340 (or during a first iteration of the same) the method 300 may comprise performing a sensitivity analysis of the heat distribution model in a step 390.

The sensitivity analysis 390 may be performed to determine a range of values for the parameters for the heat dissipation model that enable the sample thermal profiles to be produced. This identified range of values may be used to limit the bounds of any modification made to the heat distribution model (e.g. in step 344).

The sensitivity analysis 390 may also or otherwise be used to identify which parameters of the heat dissipation model have the largest influence on the predicted thermal profile (to identify which parameters can be modified to adjust the predicted thermal profile, and thereby cause the heat dissipation model to converge more quickly).

The outcome of the sensitivity analysis can be used to define the bounds of parameters for the heat dissipation model and/or define which parameters of the heat dissipation model are modified (and/or to what extent the parameters are modified) in step 344.

The process 300 may be adapted to facilitate a multi-tissue heat dissipation model, i.e. heat dissipation model that is adaptive to different types of tissue. In such examples, it will be apparent that a sample thermal profile provides a sample thermal profile in a particular type of tissue.

In some examples, the heat dissipation model provides one or more different sub-models that separately act as the heat distribution model for different types of tissue. Sample thermal profiles may be filtered (to identify the tissue associated with the sample thermal profile), and used to separately generate or modify the appropriate sub-model.

In other examples, the heat dissipation model uses tissue information to modify a generic output of the heat dissipation model (e.g. to perform scaling of the determined heat distribution). Thus, tissue information may act as a variable parameter for the heat dissipation model.

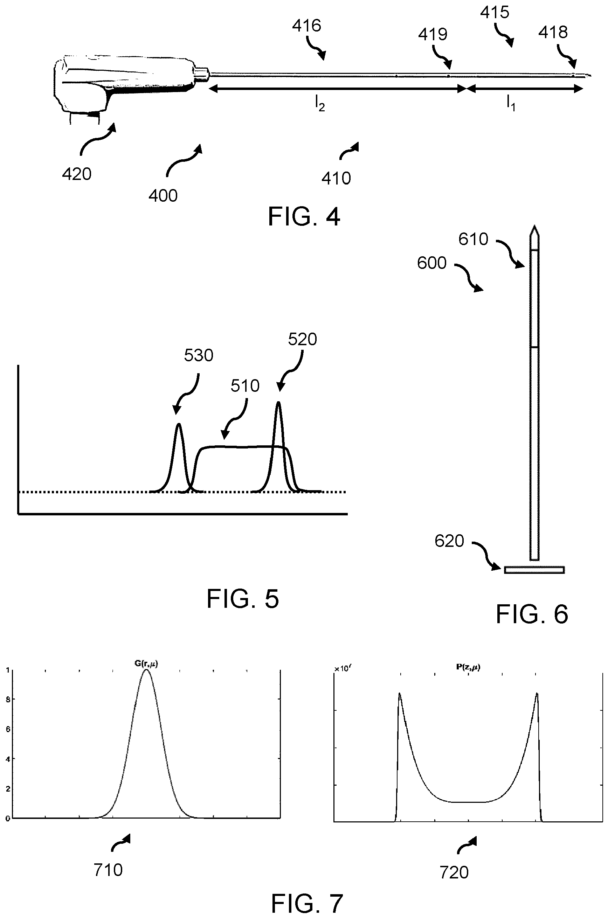

Fig. 4 illustrates an ablation device 400, which acts a target ablation device for an embodiment of the invention. The following description describes an exemplary mechanism for adding a heat distribution model for the target ablation device 400 to a heat distribution model database.

The ablation device 400 comprises a probe 410 and a control element 420, in the manner of the ablation device previous described. The ablation device 400 is associated with a number of fixed properties, some of which are visible in Fig. 4.

In particular, the probe 410 comprises a heat emitting element 415, having a first length li. The probe further comprising a cooling element 416 having a second length $l_2$. The first and second lengths, identities of the elements 415, 416 and/or the position of the elements 415, 416 may form fixed properties for the ablation device 400.

Other elements of the ablation device 400 may form or define further fixed properties. For example, it may be known that the ablation probe 400 has a first hotspot 418 close to its tip, a second hotspot 419 set apart from the emitting element and/or that the cooling element has a lower cooling temperature proximate to the heat emitting element 415.

This information about the ablation device 400 can be translated into parametric shapes and ranges for the values of the fixed properties, as illustrated in Fig. 5.

Fig. 5 conceptually illustrates a heat distribution model using shapes that illustrate a distribution of heat about the ablation probe with respect to a first plane. In Fig. 5, the x-axis (horizontal) may represent distance along the probe 410 and the y-axis (vertical) may represent a magnitude of heat output by the ablation device 400 with respect to the position along the probe.

The shapes may, for example, be scaled based on values of variable parameters, for example, power and time of operation.

The heat emitting element 415 may be represented by a first heat distribution sub-model 510, which indicates a heat output by the heat emitting element 415. For the behavior of heat across the heat emitting element, it is possible to rely on analytic solutions of simplified versions of the heat produced by MW antennas, e.g. as set out in L. Zhu, L. X. Xu, and N. Chencinski, "Quantification of the 3-D electromagnetic power absorption rate in tissue during transurethral prostatic microwave thermotherapy using heat transfer model," IEEE Trans. Biomed. Eng., vol. 45, no. 9, pp. 1163-1172, 1998. Alternatively, we can define an analytic function to describe the heat decay, using for example, a Gaussian function, a rational function or the like.

A first hotspot 418 may be represented by a second heat distribution sub-model 520. A second hotspot may be represent by a third heat distribution sub-model 530. A similar approach may be used

Thus, it is clear how the fixed properties (e.g. positions and identities of the elements of the ablation device) can be used to define sub-models of the heat distribution of the ablation device.

The three heat distribution sub-models are parametrized with respect to a scaling factor, and indicate a predicted decay of the heat for each element identified.

The combination of the three heat distribution sub-models provide a combined heat distribution model. Parameters of the heat distribution model can be modified if an accuracy of the heat distribution model does not meet predetermined criteria, as previously explained.

Figs. 6 and 7 illustrates another scenario in which the invention can be employed.

Fig. 6 illustrates an ablation device 600, which here comprises a radiofrequency (RF) ablation device, that acts as the target ablation device. A heat distribution model for the ablation device can be determined based on properties of the ablation device 600 and sample thermal profiles for the ablation device.

Fixed properties of the ablation device 600 include the modality of the ablation device (here: RF), a type of the ablation device (here: monopolar, e.g. rather than bipolar or umbrella) and the positions of the source 610 and sink 620 of the electric current.

This information may be obtained (following the process previously described) and used to generate shape functions that approximate the modality specific heat, i.e. generate sub-models of heat distribution.

Fig. 7 illustrates exemplary sub-models for the ablation device 600. A first sub-model 710 approximates the heat in a radial direction, using a Gaussian function. A second sub-model 720 approximates the heat in the longitudinal direction, using a polynomial and sigmoid function. The shape and magnitude of the sub-models is dependent upon characteristics the fixed properties of the ablation device 600.

The heat distribution model around the monopolar probe is the product of these two functions. This heat distribution model can then undergo an iterative modification process based on the obtained sample thermal profiles.

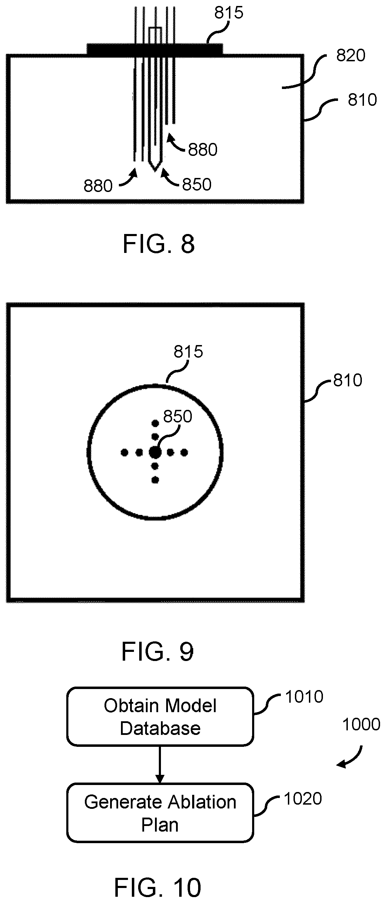

Figs. 8 and 9 illustrates an experimental setup 800 that can be used for obtaining sample thermal profiles for use in the present disclosure. Fig. 8 is a side view of the experimental set up, and Fig. 9 provides a top view of the experimental set up.

Use of an experimental set up to generate sample thermal profiles can help improve the accuracy of the heat distribution model for a target ablation device, especially in cases where the information provided by the manufacturer of the target ablation device is sparse.

The experimental setup 800 comprises a box 810 (e.g. formed of acrylic) filled with water 820, or tissue-mimicking gel (e.g. comprising a suspension of glycerol in water). The box 810 is secured with a lid 815, comprising holes dedicated for the ablation device 850 and one or more thermocouples 880 that are inserted in the box. The thermocouples can be inserted in different depths, so that multiple measurements along the emitting part of the ablation device can be taken. Additional thermocouples (not illustrated) can be inserted in positions further away from the probe.

Positions and temperature measurements of the thermocouples 880 and the recorded during a sample operation of the ablation device 850, and are subsequently used to generate a sample thermal profile for use in the methods of the present disclosure.

Sample thermal profiles generated in this manner can improve the fitting of the heat dissipation model and thereby improve the accuracy of the heat dissipation model.

Fig. 10 illustrates a method 1000 according to another embodiment.

The method 1000 comprises a step 1010 obtaining a heat distribution model database, comprising at least one entry created or updated using the methods previously described.

The method 1000 further comprises a step 1020 of generating an ablation plan for a subject using the obtained heat distribution model database. Methods for generating an ablation plan using an appropriate heat distribution model will be apparent to the skilled person and may comprise, for example, automatically generating an ablation plan to achieve a desired ablation goal (e.g. ablation of a desired region). This may comprise automatic selection and positioning of ablation device, and determining of appropriate values for the variable parameters of the ablation device(s) to ablate a desired volume.

The present disclosure identifies a number of possible fixed/variable properties for an ablation device, however, the skilled person would be readily capable of identifying other possible fixed/variable properties for an ablation device.

As a simple example, a fixed property of the ablation device may be a label or identity of the ablation device. Some further examples of fixed properties of the ablation device include the modality (e.g. MW, RF or cryo) of the ablation device, a thickness of the ablation device, cooling properties of the ablation device, a material of the ablation device, a size (e.g. length and/or width) of one or more elements of the ablation device (such as a size of the probe), a shape of the ablation device, a shape of the tip of the ablation device, geometric measurements of the ablation device, a type of one or more elements the ablation device (e.g. for a MW ablation device: a shape or type of the antenna of the ablation device; for a cryo device the presence or absence of a impinging gas jet or a type of refrigerant used).

Some possible fixed properties are dependent upon the modality of the ablation device.

For example, for an RF ablation device, the fixed properties may include the positions of the source and/or sink of electric current, which is usually readily identifiable on the ablation device. The present disclosure recognizes that the location of the sink of electric current (e.g. ground pad) affects the heat distribution, e.g. it may extend along the device axis towards the ground pad may not being perfectly symmetric around the device axis (if the ground pad is not centrally located).

As another example, for a cryo ablation device, a fixed property may include, the presence or absence of an impinging gas jet, the element makeup of the refrigerant (e.g. whether Argon, $NO_2$, liquid nitrogen or another refrigerant is used), fixed characteristics of a balloon (which is filled with refrigerant), and so on.

Some further examples of variable properties for an ablation device include more variable parameters of the target ablation device comprise: a length of operation of the target ablation device; a power provided to the ablation device; a power profile of the target ablation device over time; a position of the target ablation device within a subject; a tissue type surrounding the target ablation device during operation of the target ablation device; a tissue condition of the target ablation device during operation of the target ablation device; and an operating mode of the target ablation device.

For a cryo ablation device, a variable property may include flow rate of the refrigerant (e.g. Argon flow rate) and/or other gas (e.g. helium for inducing a thaw), a size of the active region of the needle and/or other suitable variable properties.

For a RF ablation device, a variable property may include a magnitude of a power applied by the RF ablation device, a frequency of RF emissions by the RF ablation device and/or other suitable variable properties.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A computer-implemented method for creating or updating an entry for a target ablation device in a heat distribution model database, each entry mapping an ablation device to a heat distribution model of the ablation device, the computer-implemented method comprising:

   - obtaining values for one or more fixed properties of the target ablation device;
   - obtaining one or more sample thermal profiles, each sample thermal profile representing the thermal profile that results from operating the target ablation device according to predetermined values of one or more variable parameters of the target ablation device;
   - obtaining, using the values of the one or more fixed properties, a heat distribution model of the target ablation device, the heat distribution model enabling the estimation of a thermal profile that results from operating the target ablation device according to different possible values for one or more variable parameters;
   - for each sample thermal profile, using the heat distribution model and the predetermined values of the one or more variable parameters of the sample thermal profile to generate a predicted thermal profile;
   - determining an accuracy of the heat distribution model by comparing each predicted thermal profile to the corresponding sample thermal profile;
   - in response to the accuracy of the heat distribution model failing to meet predetermined criteria, modifying the heat distribution model; and
   - in response to the accuracy of the heat distribution model meeting predetermined criteria, creating or updating an entry for the target ablation device in the heat distribution model database using the heat distribution model.

2. The computer-implemented method of claim 1, wherein each variable parameter represents either a variable parameter of the target ablation device used when producing the sample thermal profile or a property of an environment in which the target ablation device is operated when producing the sample thermal profile.

3. The computer-implemented method of any of claims 1 or 2, wherein:

the step of obtaining values for one or more fixed properties comprises obtaining a user input defining values for at least one of the one or more fixed properties; and/or

the step of obtaining one or more sample thermal profiles comprises obtaining one or more sample thermal profile not previously associated with the target ablation device.

4. The computer-implemented method of any of claims 1 to 3, wherein the step of generating a heat distribution model for the target ablation device comprises:

- processing the heat distribution model database using the values of the one or more fixed properties of the target ablation model to identify a similar heat distribution model, the similar heat distribution model having similar values for the one or more fixed properties as the corresponding values of the target ablation model; and
- using the heat distribution model of the similar target model as the heat distribution model for the target ablation device.

5. The computer-implemented method of any of claims 1 to 3, wherein the step of generating a heat distribution model for the target ablation device comprises:

- obtaining a heat distribution sub-model database, each entry in the heat distribution sub-model database providing a heat distribution sub-model for different values of one or more potential fixed properties for an ablation device;
- processing the heat distribution sub-model database using the values of the one or more fixed properties of the target ablation model to identify one or more heat distribution sub-models for the target ablation device; and
- processing the identified one or more heat distribution sub-models to generate a heat distribution model for the target ablation device.

6. The computer-implemented method of any of claims 1 to 5, wherein the one or more variable parameters of the target ablation device comprise: a length of operation of the target ablation device; a power provided to the ablation device; a power profile of the target ablation device over time; a position of the target ablation device within a subject; a tissue type surrounding the target ablation device during operation of the target ablation device; a tissue condition of the target ablation device during operation of the target ablation device; an operating mode of the target ablation device.

7. The computer-implemented method of any of claims 1 to 6, wherein the step of, in response to the accuracy of the heat distribution model failing to meet predetermined criteria, modifying the heat distribution model comprises performing iterative steps of:

- modifying the heat distribution model;
- for each sample thermal profile, using the modified heat distribution model and the predetermined values of the one or more variable parameters of the sample thermal profile to regenerate a predicted thermal profile; and
- determining an accuracy of the heat distribution model by comparing each predicted thermal profile to the corresponding sample thermal profile,
- wherein the iterative steps are performed until the accuracy of the heat distribution model meets the predetermined criteria.

8. The computer-implemented method of any of claims 1 to 7, wherein the step of modifying the heat distribution model comprises using an optimization algorithm, such as a least squares fitting algorithm, a genetic algorithm, a machine-learning approach, a gradient descent approach and/or a combinational optimization approach to iteratively modify the heat distribution model until the accuracy of the heat distribution model meets the predetermined criteria.

9. The computer-implemented method of any of claims 1 to 8, wherein the heat distribution model comprises one or more thermal functions that estimate a distribution of heat about the target ablation device during operation of the target ablation device based on one or more variable parameters.

10. The computer-implemented method of any of claims 1 to 9, wherein the step of obtaining one or more sample thermal profiles comprises obtaining one or more sample thermal profiles generated by using the target ablation device in an experimental set up to generate one or more sample thermal profiles for different values of the one or more variable parameters.

11. The computer-implemented method of any of claims 1 to 10, further comprising a step of performing a sensitivity analysis on the obtained heat distribution model.

12. The computer-implemented method of claim 11, wherein the step of modifying the heat distribution model is dependent upon an outcome of the sensitivity analysis.

13. A computer-implemented method of generating an ablation plan for a subject, comprising generating an ablation plan for a subject using a heat distribution model database comprising at least one entry created or updated using the method of any of claims 1 to 12.

14. A computer program product comprising computer program code means which, when executed on a computing device having a processing system, cause the processing system to perform all of the steps of the method according to any of claims 1 to 13.

15. A processing system for creating or updating an entry for a target ablation device in a heat distribution model database, each entry mapping an ablation device to a heat distribution model of the ablation device, the processing system being configured to:

    - obtain values for one or more fixed properties of the target ablation device;
    - obtain one or more sample thermal profiles, each sample thermal profile representing the thermal profile that results from operating the target ablation device according to predetermined values of one or more variable parameters of the target ablation device;
    - generate, using the values of the one or more fixed properties, a heat distribution model of the target ablation device, the heat distribution model enabling the estimation of a thermal profile that results from operating the target ablation device according to different values for one or more variable parameters;
    - for each sample thermal profile, using the heat distribution model and the predetermined values of the one or more variable parameters of the sample thermal profile to generate a predicted thermal profile;
    - determine an accuracy of the heat distribution model by comparing each predicted thermal profile to the corresponding sample thermal profile;
    - in response to the accuracy of the heat distribution model failing to meet predetermined criteria, modify the heat distribution model; and
    - in response to the accuracy of the heat distribution model meeting predetermined criteria, create or update an entry for the target ablation device in the heat distribution model database using the heat distribution model.

FIG. 1

FIG. 2

FIG. 3

FIG. 4

FIG. 5

FIG. 6

FIG. 7

FIG. 8

FIG. 9

FIG. 10

Europäisches
Patentamt

European
Patent Office

Office européen
des brevets

# EUROPEAN SEARCH REPORT

Application Number

EP 20 21 2954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 10 359 323 B2 (KONINKLIJKE PHILIPS NV [NL]) 23 July 2019 (2019-07-23) * columns 7-8, 19, lines 42-11, 16-44; figures 1, 5 * | 1-15 | INV. A61B18/02 A61B18/12 A61B18/18 A61B34/00 A61B34/10 |
| A | WO 2015/128145 A1 (KONINKL PHILIPS NV [NL]) 3 September 2015 (2015-09-03) * page 11, line 23 - page 14, line 24; figures 2, 5, 6 * | 1-15 | |
| A | EP 2 684 534 A1 (COVIDIEN LP [US]) 15 January 2014 (2014-01-15) * paragraph [0109] - paragraph [0112]; figure 5 * | 1-15 | ADD. A61B18/14 A61B18/00 |
| A | EP 2 016 919 A1 (COVIDIEN AG [CH]) 21 January 2009 (2009-01-21) * paragraph [0089] - paragraph [0092]; figure 17 * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 21 May 2021 | Tomelleri, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 2954

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2021

| Patent document cited in search report | | Publication date | Patent family member(s) | | Publication date |
|---|---|---|---|---|---|
| US 10359323 | B2 | 23-07-2019 | CN | 105358065 A | 24-02-2016 |
| | | | EP | 3016593 A1 | 11-05-2016 |
| | | | JP | 6392864 B2 | 19-09-2018 |
| | | | JP | 2016527934 A | 15-09-2016 |
| | | | US | 2016131540 A1 | 12-05-2016 |
| | | | WO | 2015000721 A1 | 08-01-2015 |
| WO 2015128145 | A1 | 03-09-2015 | CN | 106061417 A | 26-10-2016 |
| | | | EP | 3113702 A1 | 11-01-2017 |
| | | | JP | 6659560 B2 | 04-03-2020 |
| | | | JP | 2017512090 A | 18-05-2017 |
| | | | US | 2017014191 A1 | 19-01-2017 |
| | | | WO | 2015128145 A1 | 03-09-2015 |
| EP 2684534 | A1 | 15-01-2014 | EP | 2684534 A1 | 15-01-2014 |
| | | | US | 2014018793 A1 | 16-01-2014 |
| | | | US | 2016066985 A1 | 10-03-2016 |
| EP 2016919 | A1 | 21-01-2009 | AU | 2008203166 A1 | 05-02-2009 |
| | | | CA | 2637559 A1 | 16-01-2009 |
| | | | EP | 2016919 A1 | 21-01-2009 |
| | | | EP | 2401979 A2 | 04-01-2012 |
| | | | ES | 2392277 T3 | 07-12-2012 |
| | | | JP | 5371303 B2 | 18-12-2013 |
| | | | JP | 2009018169 A | 29-01-2009 |
| | | | US | 2008015664 A1 | 17-01-2008 |

EPO FORM P0459

For more details about this annex : see Official Journal of the European Patent Office, No. 12/82

## REFERENCES CITED IN THE DESCRIPTION

**Patent documents cited in the description**

- WO 2019145211 A1

**Non-patent literature cited in the description**

- **F.C. HENRIQUES ; A.R. MORITZ.** Studies of thermal injury: I. the conduction of heat to and through skin and the temperatures attained therein. *The American journal of pathology,* 1947, vol. 23 (4), 530-549
- **A.R. MORITZ ; F.C. HENRIQUES.** Studies of thermal injury: II. the relative importance of time and surface temperature in the causation of cutaneous burns. *The American journal of pathology,* 1947, vol. 23 (5), 695-720
- **STEPHEN A. ; WILLIAM C. DEWEY.** Thermal dose determination in cancer therapy. *International Journal of Radiation Oncology Biology Physics,* 1984, vol. 10.6, 787-800
- **L. ZHU ; L. X. XU ; N. CHENCINSKI.** Quantification of the 3-D electromagnetic power absorption rate in tissue during transurethral prostatic microwave thermotherapy using heat transfer model. *IEEE Trans. Biomed. Eng.,* 1998, vol. 45 (9), 1163-1172